(19) Europäisches Patentamt
European Patent Office
Office européen des brevets

(11) EP 0 859 766 B1

(12) **EUROPÄISCHE PATENTSCHRIFT**

(45) Veröffentlichungstag und Bekanntmachung des Hinweises auf die Patenterteilung:
**16.05.2001 Patentblatt 2001/20**

(21) Anmeldenummer: **96902871.1**

(22) Anmeldetag: **09.02.1996**

(51) Int Cl.[7]: **C07D 263/24**, A61K 31/42

(86) Internationale Anmeldenummer:
**PCT/DE96/00259**

(87) Internationale Veröffentlichungsnummer:
**WO 97/15561 (01.05.1997 Gazette 1997/19)**

(54) **CHIRALE METHYLPHENYLOXAZOLIDINONE**

CHIRAL METHYL PHENYL OXAZOLIDINONES

METHYLPHENYLOXAZOLIDINONES CHIRALES

(84) Benannte Vertragsstaaten:
**AT BE CH DE DK ES FR GB GR IE IT LI LU MC NL PT SE**

(30) Priorität: **20.10.1995 DE 19540475**

(43) Veröffentlichungstag der Anmeldung:
**26.08.1998 Patentblatt 1998/35**

(73) Patentinhaber: **SCHERING AKTIENGESELLSCHAFT**
**13353 Berlin (DE)**

(72) Erfinder:
- **LAURENT, Henry**
  **D-13467 Berlin (DE)**
- **OTTOW, Eckhard**
  **D-12248 Berlin (DE)**
- **KIRSCH, Gerald**
  **D-14199 Berlin (DE)**
- **WACHTEL, Helmut**
  **D-14057 Berlin (DE)**
- **SCHNEIDER, Herbert**
  **D-10707 Berlin (DE)**
- **FAULDS, Daryl**
  **Mill Valley, CA 94941 (US)**
- **DINTER, Harald**
  **San Rafael, CA 94903 (US)**

(56) Entgegenhaltungen:
**EP-A- 0 270 482** **WO-A-86/02268**
**US-A- 4 186 129**

- **CHEMICAL ABSTRACTS, vol. 122, no. 13, 27.März 1995 Columbus, Ohio, US; abstract no. 151229v, SCHNEIDER ET AL: "Discriminative stimulus properties of the stereoisimers of the phosphodiesterase inhibitor rolipram" Seite 64; Spalte 2; XP002005537 & PHARMACOL BIOCEM BEHAV., Bd. 50, Nr. 2, 1995, Seiten 211-217,**
- **CHEMICAL ABSTRACTS, vol. 113, no. 17, 22.Oktober 1990 Columbus, Ohio, US; abstract no. 145263z, SCHMIECHEN: "Close correlation between behavioral response and binding in vivo for inhibitors of the rolipram-sensitive phosphodiesterase" Seite 62; Spalte 2; XP002005538 & PSYCHOPHARMACOLOGY, Bd. 102, Nr. 1, 1990, Seiten 17-20,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 859 766 B1

Printed by Jouve, 75001 PARIS (FR)

## Beschreibung

Die Erfindung betrifft *(R)*-(--)-Methylphenyloxazolidinon-Derivate, das Verfahren zu deren Herstellung und deren Verwendung als Arzneimittel.

Aus dem US-Patent 4.186.129 ist bekannt, daß Phenyloxazolidinon-Derivate phosphodiesterasehemmende Eigenschaften besitzen und darüberhinaus zentraldepressiv, anti-dopaminerg, antinoziceptiv und antikonvulsiv wirken. Ferner wird in EP-0198919 beschrieben, daß Phenyloxazolidinone bei topischer Applikation antiinflammatorische Eigenschaften besitzen und in EP-0270482 wird die gute neuropsychotrope Wirksamkeit von Phenyloxazolidinonen offenbart.

In diesen Publikationen wird nur erwähnt, daß die Auftrennung des Racemats in die Antipoden mit den üblichen Methoden erfolgen kann, ohne daß die Enantiomeren dargestellt und deren pharmakologische Aktivität untersucht bzw. die Reinheit der erhaltenen Verbindungen festgestellt wurde. Um die Nebenwirkungen von Arzneimitteln herabzusetzen, ist es wünschenswert, eine einheitliche Wirksubstanz zu verabreichen, die in geringen Dosierungen angewendet werden kann.

Es wurde nun gefunden, daß R-konfigurierte Methylphenyloxazolidinon-Derivate besonders wirksam sind und zur Verwendung als Arzneimittel besser geeignet sind als das Racemat.

Die Erfindung betrifft *(R)*-(--)-Methylphenyloxazolidinone der Formel I,

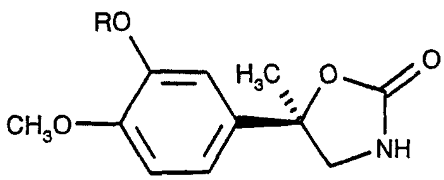

(I)

worin

R einen Kohlenwasserstoffrest mit bis zu 5 C-Atomen bedeutet.

Als Kohlenwasserstoffrest sei beispielsweise Ethyl, Propyl, Isobutyl, Isobutenyl, Butyl, Cyclobutyl und Cyclopentyl genannt.

Die Verbindungen der Formel I inhibieren auch die TNF-Produktion und sind daher zur Behandlung von Krankheiten geeignet, die über die Aktivierung von TNF vermittelt werden.

Unter Krankheiten, die durch TNF vermittelt werden, sind sowohl Krankheiten zu verstehen, die durch Produktion von TNF ausgelöst werden wie auch Krankheiten, bei denen durch TNF andere Cytokine wie beispielsweise Il-1 oder Il-6 beeinflußt werden.

Unter TNF ist sowohl TNF-$\alpha$ als auch TNF-$\beta$ zu verstehen, die beide durch die Verbindungen der Formel I antagonisiert werden. Bevorzugt wird TNF-$\alpha$ inhibiert.

Die Verbindungen der Formel I eignen sich daher zur Herstellung eines pharmazeutischen Präparates, das zur Behandlung und Prophylaxe von Erkrankungen in Lebewesen verwendet wird, die durch Stimulation von TNF ausgelöst werden. Als Erkrankungen, die durch excessive oder unregulierte TNF-Stimulation beeinflußt werden sind beispielsweise allergische und inflammatorische Erkrankungen, Autoimmunerkrankungen, pulmonare Erkrankungen, infektiöse Erkrankungen und Knochenresorptionserkrankungen bekannt wie Rheumatoide Arthritis, Rheumatoide Spondylitis, Osteoarthritis, Gicht, Sepsis, septischer Schock, Endotoxin-Schock, Gramnegative Sepsis, Toxisches Schocksyndrom, ARDS (Akutes Atemnotsyndrom), Pulmonare Sarkoidose, Asthma, Silikose, Kachexie, Colitis ulcerosa, Morbus Crohn, Osteoporose, Organschädigung nach Reperfusion, inflammatorische Erkrankungen des ZNS wie Cerebrale Malaria, Multiple Sklerose, Panencephalitis, Infektionserkrankungen wie AIDS, Rinderwahnsinn, inflammatorische Erkrankungen der Haut wie Urticaria, Psoriasis, Atopische Dermatitis, Kontaktdermatitis, Lupus Erythematosus sowie Diabetes Insipidus, Neuroprotection z. B. bei Morbus Parkinson, Demenz beispielsweise nach Multiinfarkten und Schlaganfall.

Die Wirksamkeit der Verbindungen der Formel I in den genannten Indikationen kann durch entsprechende übliche pharmakologische Teste gezeigt werden.

Die neuen *(R)*-(--)-Methylphenyloxazolidinone können aus dem Racemat durch Chromatographie an chiralen Säulen oder über Diastereomeren mit optisch aktiven Hilfsstoffen erhalten werden. Als optisch aktiver Hilfsstoff ist beispielsweise *(R)*-1-(1-Naphthyl)-ethylisocyanat geeignet, das die Herstellung der optisch aktiven Verbindung auf einfachem Weg in guten Ausbeuten und hoher Reinheit ermöglicht. Die Umsetzung wird in inerten Lösungsmitteln wie Toluol. Benzol u. a. oder deren Gemischen in Gegenwart einer organischen Base, beispielsweise einem tertiären Amin wie Triethylamin bei erhöhter Temperatur bzw. Siedetemperatur des Reaktionsgemisches vorgenommen. Das erhaltene Gemisch der diastereomeren Allophanate wird durch Chromatographie an Silicagel quantitativ in die Komponenten aufgetrennt. Die separierten diastereomeren Allophanate werden anschließend durch Behandlung mit Basen beispielsweise mit Alkalialkoholaten in polaren Lösungsmitteln in die optisch aktiven Methylphenyloxazolidinone aufgespalten. Als polare Lösungsmittel sind beispielsweise cyclische und acyclische Ether wie Tetrahydrofuran, Dioxan, Diethylether geeignet. Zweckmäßigerweise wird die Umsetzung unter Inertgas durchgeführt.

Die Erfindung umfaßt auch das Verfahren zur Herstellung der Verbindungen der Formel I dadurch, daß man deren Racemat mit einem optisch aktiven Hilfsstoff in das Diastereomerengemisch überführt und anschließend den optisch aktiven Hilfsstoff abtrennt oder deren Racemat an chiralen Säulen chromatographiert. Die Herstellung der Verbindungen der Formel I kann auch durch Trennung von *(R,S)*-5-(3-Benzyloxy-4-methoxyphenyl)-5-methyl-2-oxazolidinon beispielsweise durch Chromatographie und anschließender Abspaltung der Benzylgruppe und Veretherung erfolgen. Die Abspaltung der Benzylgruppe erfolgt beispielsweise durch Hydrierung in Gegenwart eines Katalysators wie beispielsweise Palladium auf einem geeigneten Träger in inerten Lösungsmitteln wie Ethylacetat. Die anschließende Veretherung des Hydroxyderivates erfolgt in Gegenwart von Basen mit einem reaktiven Derivat wie Halogenid, Tosylat oder Mesylat in polaren Lösungsmitteln wie Dimethylformamid oder Alkoholen bei Temperaturen bis zum Siedepunkt des Lösungsmittels. Als Basen sind z. B. Alkaliverbindungen wie Natrium- oder Kaliumhydroxide, -carbonate, -alkoholate oder -hydride geeignet.

Enthält der Substituent R eine Doppelbindung, so kann diese in üblicher Weise zum entsprechenden Alkylderivat reduziert werden. Beispielsweise kann die Reduktion katalytisch mit Palladium/Kohle in einem inerten Lösungsmittel bei Raumtemperatur oder erhöhter Temperatur erfolgen.

Die erfindungsgemäßen Verfahren ermöglichen die Herstellung der Verbindungen der Formel I in 99%iger Reinheit.

Am Beispiel von 5-(3-Cyclopentyloxy-4-methoxyphenyl)-5-methyl-2-oxazolidinon (Verbindung 1) kann gezeigt werden, daß die optisch aktive *(R)*-(--)-Verbindung überraschenderweise die wirksame Verbindung darstellt.

Die verbesserte Wirksamkeit der neuen chiralen Methylphenyloxazolidinon-Derivate im Vergleich mit dem Racemat kann anhand der für Phosphodiesterase Typ IV (PDE IV)-Inhibitoren charakteristischen Head twitch- und Grooming-Reaktion an Ratten gezeigt werden. Das Racemat und die entsprechenden Enantiomeren wurden männlichen Wistar-Ratten intraperitoneal (i. p.) verabreicht und das Auftreten von Head twitches und Grooming während der 15. - 75. Minute nach Injektion durch Beobachtung erfaßt. Wie aus der Tabelle 1 ersichtlich ist, erwies sich das *(S)*-(+)-Enantiomer gegenüber dem Racemat als 4fach (Head twitches) bzw. 60fach (Grooming) schwächer wirksam, während das *(R)*-(--)-Enantiomer 4fach stärker (Head twitches) bzw. gleich wirksam (Grooming) im Vergleich zum Racemat war.

Tabelle 1

| **Verbindung** | **Head twitch-Test** MED i. p. [mg/kg] | **Grooming** MED i. p. [mg/kg] |
|---|---|---|
| *(R)*-(--)-1 | 0.39 | 0.1 |
| *(R,S)*-1 | 1.56 | 0.1 |
| *(S)*-(+)-1 | 6.25 | 6.25 |
| MED: Minimale effektive Dosis, d. h. die niedrigste Dosis, welche einen statistisch signifikanten Effekt bewirkt. | | |

Die Wirkung der Enantiomeren auf das Zentralnervensystem wurde durch Untersuchung des Verdrängungsvermögens von radioaktiv markiertem Rolipram von Hirnhomogenaten in vitro untersucht (Eur. J. Pharmacol., Vol. 127, 105-115 (1986)). Die $IC_{50}$-Werte (diejenige Konzentration, bei der 50 % Hemmwirkung eintritt) wurden in die Inhibitionskonstante $K_i$ umgewandelt, die nach folgender Formel berechnet wird:

$$K_i = IC_{50}/[1 + (L/K_D)],$$

worin L die Konzentration des radioaktiven Tracers und $K_D$ die Dissoziationskonstante der $^3$H-Roliprambindung, die getrennt ermittelt wird, bedeutet.

**Tabelle 2**

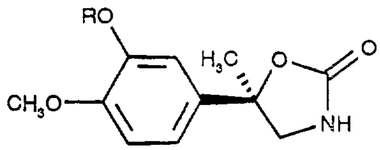

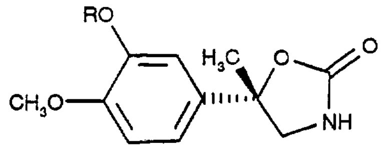

*(R)*-(–)-Isomer *(S)*-(+)-Isomer

| R | **Racemat** $K_i$ [nM] | ***(R)*-(–)-Isomer** $K_i$ [nM] | ***(S)*-(+)-Isomer** $K_i$ [nM] |
|---|---|---|---|
| Ethyl- | 0.68 | 0.33 | 20 |
| Propyl- | 0.61 | 0.24 | 16 |
| Cyclopentyl- | 0.57 | 0.34 | 3.0 |

Makrophagen und Mikrogliazellen, welche Makrophagenfunktionen im Gehirn ausüben, vermitteln die Freisetzung von TNF-α während experimenteller allergischer Encephalomyelitis (EAE). Werden Makrophagen stimuliert beispielsweise durch Lipopolysaccharid (LPS), erfolgt eine Sekretion von TNF-α in vitro und in vivo innerhalb von Stunden.

Eine murine Makrophagen Zell-Linie (RAW 264) wurde 30 Minuten preinkubiert in Gegenwart und in Abwesenheit von verschiedenen Konzentrationen von PDE-IV-Inhibitoren und anschließend mit LPS (10 ng/ml) stimuliert. 18 Stunden nach Stimulation wurde das Kulturmedium entfernt und die TNF-α-Freisetzung mit einem spezifischen Elisa-Test gemessen.

Der Test ist von verschiedenen Firmen erhältlich, unter anderem von der Firma British Biotechnology, Genzyme, und wird durchgeführt, wie es vom Hersteller beschrieben wird.

Die verbesserte TNF-Inhibition der neuen chiralen Methylphenyloxazolidinonderivate im Vergleich mit dem Razemat zeigt am Beispiel von 5-(3-Propoxy-4-methoxyphenyl)-5-methyl-2-oxazolidinon (Verbindung 2) die Tabelle 3:

Tabelle 3

| **Verbindung** | **$IC_{50}$ [μM]** |
|---|---|
| (*RS*)-2 | 0.50 |
| *(R)*-(--)-2 | 0.25 |
| *(S)*-(+)-2 | 2.50 |

Der Tabelle ist zu entnehmen, daß das (--)-Enantiomer doppelt so wirksam ist wie das Razemat und 10fach stärker wirksam als das (+)-Enantiomer.

Da die neuen Verbindungen der Formel I sich nicht nur durch gesteigerte Wirksamkeit auszeichnen, sondern auch durch geringe Nebenwirkungen und verminderte Toxizität, ist die Verwendung von optisch aktiven *(R)*-(--)-Methylphenyloxazolidinonen zur Herstellung von Arzneimitteln besonders günstig.

Die Mittel werden nach üblichen Verfahren hergestellt, indem man den Wirkstoff mit geeigneten Träger-, Hilfs- und/oder Zusatzstoffen in die Form eines pharmazeutischen Präparates bringt, das für die enterale oder parenterale Applikation geeignet ist. Die so erhaltenen Zubereitungen können als Arzneimittel in der Human- oder Veterinärmedizin eingesetzt werden. Die Applikation kann oral oder sublingual als Feststoff in Form von Kapseln oder Tabletten oder als Flüssigkeit in Form von Lösungen, Suspensionen, Elexieren, Aerosolen oder Emulsionen oder rektal in Form von Suppositorien oder in Form von gegebenenfalls auch subcutan intramuskulär oder intravenös anwendbaren Injektionslösungen oder topisch oder intrathekal erfolgen. Als Hilfsstoffe für die gewünschte Arzneimittelformulierung sind die dem Fachmann bekannten inerten organischen und anorganischen Trägermaterialien geeignet wie z. B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form, z. B. als Tabletten, Dragees, Suppositoren, Kapseln oder in flüssiger Form, z. B. als Lösungen, Suspensionen oder Emulsionen vorliegen.
Als Trägersysteme können auch grenzflächennahe Hilfsstoffe wie Salze, Gallensäuren oder tierische oder pflanzliche Phospholipide und deren Mischungen sowie Liposome oder deren Bestandteile verwendet werden.
Für die orale Anwendung sind insbesondere Tabletten, Dragees oder Kapseln mit Talkum und/oder Kohlenwasserstoffträger oder -binder, wie z. B. Lactose, Mais- oder Kartoffelstärke, geeignet. Die Anwendung kann auch in flüssiger Form erfolgen, wie z. B. als Saft, dem gegebenenfalls Süßstoff beigefügt wird.
Die Verbindungen der Formel I werden in Dosierungen angewendet, die ausreichend sind, die TNF-Produktion auf normale oder geringere Werte herabzusetzen.
Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0.1 - 25 mg, bevorzugt 0.5 - 5 mg, wobei die Dosis als einmal zu verabreichende Einzeldosis oder unterteilt in zwei oder mehreren Tagesdosen gegeben werden kann.
Soweit die Herstellung der Ausgangsverbindungen nicht beschrieben wird, sind diese aus den genannten Publikationen bekannt oder analog zu bekannten Verbindungen oder hier beschriebenen Verfahren herstellbar.
Die nachfolgenden Beispiele sollen das erfindungsgemäße Verfahren erläutern.

**Ausgangsverbindungen:**

***(R,S)*-2-(3-Cyclopentyloxy-4-methoxyphenyl)-2-hydroxy-1-propylamin**

16.9 g 3-Cyclopentyloxy-4-methoxy-acetophenon werden unter Erwärmen in 12.5 ml Trimethylsilylcyanid gelöst. Nach Zugabe von 700 mg Zinkiodid tritt eine starke Wärmetönung auf; danach wird auf 20 °C abgekühlt und 30 Minuten unter Stickstoff gerührt. Die Reaktionsmischung wird mit 100 ml Tetrahydrofuran versetzt und innerhalb von 20 Minuten in eine Lösung von 4.4 g Lithiumalanat in 100 ml Tetrahydrofuran getropft. Nach weiteren 30 Minuten werden 100 ml einer gesättigten Kaliumnatriumtartrat-Lösung vorsichtig hinzugegeben. Es bildet sich eine breiartige Masse, von der sich die Tetrahydrofuranphase abdekantieren läßt. Der breiige Rückstand wird siebenmal mit je 100 ml Diethylether extrahiert, die Extrakte werden zusammen mit der Tetrahydrofuranphase im Vakuum eingedampft. Der Rückstand wird in 300 ml Ethylacetat gelöst und dreimal mit je 50 ml 2 N Chlorwasserstoffsäure ausgezogen. Die vereinigten sauren Extrakte werden mit 4 N Natronlauge auf pH 13 eingestellt und sechsmal mit je 100 ml Diethylether extrahiert. Die Etherauszüge werden über Natriumsulfat getrocknet und im Vakuum eingedampft. Man erhält als Rückstand 16.4 g *(R,S)*-2-(3-Cyclopentyloxy-4-methoxyphenyl)-2-hydroxy-1-propylamin mit einem Schmelzpunkt von 82 °C.

***(R,S)*-5-(3-Cyclopentyloxy-4-methoxyphenyl)-5-methyl-2-oxazolidinon**

Eine Lösung von 16.4 g *(R,S)*-2-(3-Cyclopentyloxy-4-methoxyphenyl)-2-hydroxy-1-pro-pylamin in 150 ml Tetrahydrofuran wird mit 10.2 g N,N'-Carbonyldiimidazol versetzt und 3 Stunden beim Raumtemperatur gerührt. Das Reaktionsgemisch wird im Vakuum eingedampft, der Rückstand wird in 500 ml Ethylacetat gelöst und die Lösung zweimal mit je 50 ml 2 N Chlorwasserstoffsäure und anschließend mit Wasser gewaschen, getrocknet und im Vakuum eingedampft. Als Rückstand erhält man 17.7 g *(R,S)*-5-(3-Cyclopentyloxy-4-methoxyphenyl)-5-methyl-2-oxazolidinon. Schmelzpunkt 83.5 °C.

***(R,S)*-2-(4-Methoxy-3-propoxyphenyl)-2-hydroxy-1-propylamin**

Eine Mischung von 30 g 4-Methoxy-3-propoxy-acetophenon und 25 ml Trimethylsilylcyanid wird mit 1.4 g Zinkiodid versetzt und 4 Stunden auf 110 °C erhitzt. Nach dem Abkühlen wird das Reaktionsgemisch mit 200 ml Tetrahydrofuran verdünnt, tropfenweise mit einer Suspension von 8.0 g Lithiumalanat in 200 ml Tetrahydrofuran versetzt und eine Stunde zum Sieden erhitzt. Nach dem Abkühlen auf 4 °C wird mit 750 ml Diethylether verdünnt und die Mischung anschließend über einen Zeitraum von 45 Minuten vorsichtig mit gesättigter Natriumhydrogencarbonatlösung bis zum Abscheiden von festem Aluminiumhydroxid versetzt. Die organische Phase wird abgetrennt und das zurückbleibende anorganische Material mit 1000 ml Diethylether gewaschen. Die vereinigten organischen Phasen werden im Vakuum eingedampft, der Rückstand in Dichlormethan aufgenommen und viermal mit je 80 ml wässriger 2 N Chlorwasserstoffsäure extrahiert. Die vereinigten sauren wässrigen Phasen werden mit wässriger 5 N Natriumhydroxidlösung auf pH 10 gebracht und nach Sättigung mit Natriumchlorid wiederholt mit Ethylacetat extrahiert. Die vereinigten Extrakte werden über Natriumsulfat getrocknet und im Vakuum eingedampft. Man erhält als Rückstand 25.0 g *(R,S)*-2-(4-Methoxy-3-propoxyphenyl)-2-hydroxy-1-propylamin mit einem Schmelzpunkt von 90 °C.

***(R,S)*-5-(4-Methoxy-3-propoxyphenyl)-5-methyl-2-oxazolidinon**

Eine Lösung von 24.0 g *(R,S)*-2-(4-Methoxy-3-propoxyphenyl)-2-hydroxy-1-propylamin in 260 ml Tetrahydrofuran wird unter Eiskühlung mit 19.4 g N,N'-Carbonyldiimdazol versetzt und anschließend 16 Stunden bei Raumtemperatur gerührt. Das Lösemittel wird im Vakuum verdampft, der Rückstand wird in 300 ml Ethylacetat gelöst und die Lösung dreimal mit je 50 ml wässriger 1 N Chlorwasserstoffsäure gewaschen. Anschließend wird die organische Phase mit Natriumhydrogencarbonat- sowie mit Natriumchloridlösung gewaschen, über Natriumsulfat getrocknet und im Vakuum eingedampft. Der ölige Rückstand von 28 g wird durch Chromatographie an einer Kieselgelsäule, mit einem Hexan-Ethylacetat-Gemisch als Eluant, gereinigt. Es resultieren 24.5 g *(R,S)*-5-(4-Methoxy-3-propoxyphenyl)-5-methyl-2-oxazolidinon. Schmelzpunkt 71 °C.

***(R,S)*-2-(3-Ethoxy-4-methoxyphenyl)-2-hydroxy-1-propylamin**

Eine Mischung von 28 g 3-Ethoxy-4-methoxy-acetophenon und 25 ml Trimethylsilylcyanid wird mit 1.4 g Zinkiodid versetzt und 4 Stunden auf 100 °C erhitzt. Nach dem Abkühlen wird das Reaktionsgemisch mit 200 ml Tetrahydrofuran verdünnt, tropfenweise mit einer Suspension von 8.0 g Lithiumalanat in 200 ml Tetrahydrofuran versetzt und eine Stunde zum Sieden erhitzt. Nach dem Abkühlen auf 4 °C wird mit 750 ml Diethylether verdünnt und die Mischung anschließend über einen Zeitraum von 45 Minuten vorsichtig mit gesättigter Natriumhydrogencarbonatlösung bis zum Abscheiden von Aluminiumhydroxid versetzt. Die organische Phase wird abgetrennt und das zurückbleibende anorganische Material mit 1000 ml Diethylether gewaschen. Die vereinigten organischen Phasen werden im Vakuum eingedampft, der Rückstand in Dichlormethan aufgenommen und viermal mit je 80 ml wässriger 2 N Chlorwasserstoffsäure extrahiert. Die vereinigten sauren wässrigen Phasen werden mit wässriger 5 N Natriumhydroxidlösung auf pH 10 gebracht und nach Sättigung mit Natriumchlorid wiederholt mit Ethylacetat extrahiert. Die vereinigten Extrakte werden über Natriumsulfat getrocknet und im Vakuum eingedampft. Man erhält als Rückstand 26.1 g *(R,S)*-2-(3-Ethoxy-4-methoxyphenyl)-2-hydroxy-1-propylamin mit einem Schmelzpunkt von 88 °C.

***(R,S)*-5-(3-Ethoxy-4-methoxyphenyl)-5-methyl-2-oxazolidinon**

Eine Lösung von 11.2 g *(R,S)*-2-(3-Ethoxy-4-methoxyphenyl)-2-hydroxy-1-propylamin in 130 ml Tetrahydrofuran wird unter Eiskühlung mit 9.7 g N,N'-Carbonyldiimdazol versetzt und anschließend 16 Stunden bei Raumtemperatur gerührt. Das Lösemittel wird im Vakuum verdampft, der Rückstand wird in 200 ml Ethylacetat gelöst und die Lösung zweimal mit je 50 ml wässriger 1 N Chlorwasserstoffsäure gewaschen. Anschließend wird die organische Phase mit Natriumhydrogencarbonat- und Natriumchloridlösung gewaschen, über Natriumsulfat getrocknet und im Vakuum eingedampft. Der ölige Rückstand von 12 g wird durch Chromatographie an einer Kieselgelsäule, mit einem Hexan-Ethylacetat-Gemisch als Eluant, gereinigt. Es resultieren 9.6 g (R,S)-5-(3-Ethoxy-4-methoxyphenyl)-5-methyl-2-oxazolidinon. Schmelzpunkt 102 °C.

**Beispiel 1**

**Darstellung und Trennung der diastereomeren Allophanate**

17.7 g *(R,S)*-5-(3-Cyclopentyloxy-4-methoxyphenyl)-5-methyl-2-oxazolidinon werden in 240 ml Toluol gelöst. Nach Zugabe von 9 ml Triethylamin und 12.8 g *(R)*-1-(1-Naphthyl)-ethylisocyanat wird die Reaktionslösung 17 Stunden unter Stickstoff zum Sieden erhitzt und anschließend im Vakuum eingedampft. Der Rückstand von 31.1. g wird an einer Silicagelsäule (Kromasil, 10 µm) mit einem Hexan-Diethylether-Gemisch (6:4) chromatographiert. Es werden 11.5 g N-[*(R)*-1-(1-Naphthyl)ethyl]-*(R)*-5-(3-Cyclopentyloxy-4-methoxyphenyl)-5-methyl-2-oxazolidinon-3-carbonsäureamid, Schmelzpunkt 124 °C, $[\alpha]_D$ = -8° ($CHCl_3$), sowie 13.5 g N-[*(R)*-1-(1-Naphthyl)ethyl]-*(S)*-5-(3-Cyclopentyloxy-4 methoxyphenyl)-5-methyl-2-oxazolidinon-3-carbonsäureamid, ölig, $[\alpha]_D$ = -41° ($CHCl_3$), eluiert.

***(R)*-(-)-5-(3-Cyclopentyloxy-4-methoxyphenyl)-5-methyl-2-oxazolidinon**

Eine Lösung von 11.0 g N-[*(R)*-1-(1-Naphthyl)ethyl]-*(R)*-5-(3-Cyclopentyloxy-4-methoxyphenyl)-5-methyl-2-oxazolidinon-3-carbonsäureamid in 230 ml Tetrahydrofuran wird unter Eiskühlung und in einer Stickstoffatmosphäre mit 2.3 g Kaliumethylat versetzt und 30 Minuten bei Raumtemperatur gerührt. Nach Zugabe von 700 ml Ethylacetat wird zweimal mit je 50 ml 2 N Chlorwasserstoffsäure und anschließend mit Wasser gewaschen, getrocknet und im Vakuum eingedampft. Das Rohprodukt von 12.3 g wird an einer Silica-gelsäule (Kromasil, 10 µm) mit einem Ethylacetat-Hexan-Gemisch (3:7) chromatographiert. Es werden 6.68 g eluiert und aus Hexan-Dichlormethan umkristallisiert. Ausbeute: 6.23 g *(R)*-(--)-5-(3-Cyclopentyloxy-4-methoxyphenyl)-5-methyl-2-oxazolidin-on. Schmelzpunkt 84 °C. $[\alpha]_D$ =

-41° ($CHCl_3$).

***(S)*-(+)-5-(3-Cyclopentyloxy-4-methoxyphenyl)-5-methyl-2-oxazolidinon**

Eine Lösung von 490 mg N-[*(R)*-1-(1-Naphthyl)ethyl]-*(S)*-5-(3-Cyclopentyloxy-4-meth-oxyphenyl)-5-methyl-2-oxazolidinon-3-carbonsäureamid in 10 ml Tetrahydrofuran wird in einer Stickstoffatmosphäre mit 90 mg Kaliumethylat versetzt und eine Stunde bei Raumtemperatur gerührt. Nach Zugabe von *50* ml Ethylacetat wird zweimal mit je 10 ml 2 N Chlorwasserstoffsäure und anschließend mit Wasser gewaschen, getrocknet und im Vakuum eingedampft. Das Rohprodukt von 470 mg wird an einer Silicagelsäule (Kromasil, 10 μm) mit einem Ethylacetat-Hexan-Gemisch (3:7) chromatographiert. Es werden 260 mg kristallines *(S)*-(+)-5-(3-Cyclopentyloxy-4-methoxyphenyl)-5-methyl-2-oxazolidinon eluiert. Schmelzpunkt 80 °C. $[\alpha]_D$ = +38° ($CHCl_3$).

**Beispiel 2**

**Darstellung und Trennung der diastereomeren Allophanate**

14.6 g *(R,S)*-5-(4-Methoxy-3-propoxyphenyl)-5-methyl-2-oxazolidinon werden in 200 ml Toluol gelöst. Nach Zugabe von 7.7 ml Triethylamin und 10.0 g *(R)*-1-(1-Naphthyl)-ethylisocyanat wird die Reaktionslösung 16 Stunden unter Stickstoff zum Sieden erhitzt. Nach dem Abkühlen auf Raumtemperatur wird im Vakuum eingedampft, der Rückstand in Ethylacetat gelöst, feste Bestandteile werden abfiltiert und die Lösung im Vakuum konzentriert. Der Rückstand wird an einer Silicagelsäule (Kromasil, 10 μm) mit einem Ethylacetat-Hexan-Gemisch (3:7) chromatographiert. Es werden 10.9 g eluiert. Nach Rekristallisieren aus Ethylacetat-Hexan erhält man 7.0 g N-[*(R)*-1-(1-Naphthyl)ethyl]-*(R)*-5-(4-Methoxy-3-propoxyphenyl)-5-methyl-2-oxazolidinon-3-carbonsäureamid. Schmelzpunkt 106 °C. $[\alpha]_D$ = -9° ($CHCl_3$). Weiterhin werden 12.4 g N-[*(R)*-1-(1-Naphthyl)ethyl]-*(S)*-5-(4-Methoxy-3-propoxyphenyl)-5-methyl-2-oxazolidinon-3-carbonsäureamid als Öl eluiert. $[\alpha]_D$ = -43° ($CHCl_3$).

***(R)*-(--)-5-(4-Methoxy-3-propoxyphenyl)-5-methyl-2-oxazolidinon**

Unter Eiskühlung wird eine Lösung von 10.0 g N-[*(R)*-1-(1-Naphthyl)ethyl]-*(R)*-5-(4-Methoxy-3-propoxyphenyl)-5-methyl-2-oxazolidinon-3-carbonsäureamid in 200 ml Tetrahydrofuran mit 2.3 g Kaliumethylat versetzt und anschließend bei Raumtemperatur 1.5 Stunden gerührt. Nach Zugabe von 400 ml Ethylacetat wird zweimal mit je 50 ml 2 N Chlorwasserstoffsäure und anschließend mit Wasser gewaschen, getrocknet und im Vakuum eingedampft. Das Rohprodukt von 8.3 g wird mit einer Mischung aus Ethylacetat und Hexan als Eluant an einer Silicagelsäule chromatographiert. Man erhält 5.3 g (R)-(--)-5-(4-Methoxy-3-propoxyphenyl)-5-methyl-2-oxazolidinon, das aus einem Ethylacetat-Hexan-Gemisch umkristallisiert wird. Ausbeute: 4.5 g. Schmelzpunkt 93 °C. $[\alpha]_D$ = -48° ($CHCl_3$).

***(S)*-(+)-5-(4-Methoxy-3-propoxyphenyl)-5-methyl-2-oxazolidinon**

Unter Eiskühlung wird eine Lösung von 13.8 g N-[*(R)*-1-(1-Naphthyl)ethyl]-*(S)*-5-(4-Methoxy-3-propoxyphenyl)-5-methyl-2-oxazolidinon-3-carbonsäureamid in 200 ml Tetrahydrofuran mit 4.8 g Kaliumethylat versetzt und anschließend bei Raumtemperatur 16 Stunden gerührt. Nach Zugabe von 400 ml Ethylacetat wird zweimal mit je 50 ml 2 N Chlorwasserstoffsäure und anschließend mit Wasser gewaschen, getrocknet und im Vakuum eingedampft. Das Rohprodukt von 16.5 g wird mit einer Mischung aus Ethylacetat und Hexan als Eluant an einer Silicagelsäule chromatographiert. Man erhält 8.3 g *(S)*-(+)-5-(4-Methoxy-3-propoxyphenyl)-5-methyl-2-oxazolidinon. Nach Kristallisation aus Hexan-Ethylacetat verbleiben 6.4 g. Schmelzpunkt 94 °C. $[\alpha]_D$ = +45° ($CHCl_3$).

**Beispiel 3**

**Darstellung und Trennung der diastereomeren Allophanate**

5.9 g *(R,S)*-5-(3-Ethoxy-4-methoxyphenyl)-5-methyl-2-oxazolidinon werden in 90 ml Toluol gelöst. Nach Zugabe von 3.3 ml Triethylamin und 4.8 g *(R)*-1-(1-Naphthyl)-ethylisocyanat wird die Reaktionslösung 25 Stunden unter Stickstoff zum Sieden erhitzt. Nach dem Abkühlen auf Raumtemperatur wird im Vakuum eingedampft, der Rückstand in Ethylacetat gelöst, feste Bestandteile werden abfiltiert und die Lösung im Vakuum konzentriert. Der Rückstand wird an einer Silicagelsäule (Kromasil, 10 μm) mit einem Ethylacetat-Hexan-Gemisch (3:7) chromatographiert. Es werden 4.55 g N-[(R)-1-(1-Naphthyl)ethyl]-*(R)*-5-(3-ethoxy-4-methoxyphenyl)-5-methyl-2-oxazolidinon-3-carbon-säureamid eluiert. Schmelzpunkt 112 °C. $[\alpha]_D$ = -12° ($CHCl_3$). Weiterhin werden 4.4 g N-[*(R)*-1-(1-Naphthyl)ethyl]-*(S)*-5-(3-ethoxy-4-methoxyphenyl)-5-methyl-2-oxazolidinon-3-carbonsäureamid als Öl eluiert. $[\alpha]_D$ = -39° ($CHCl_3$).

***(R)*-(--)-5-(3-Ethoxy-4-methoxyphenyl)-5-methyl-2-oxazolidinon**

Unter Eiskühlung wird eine Lösung von 7.3 g N-[*(R)*-1-(1-Naphthyl)ethyl]-*(R)*-5-(3-eth-oxy-4-methoxyphenyl)-5-methyl-2-oxazolidinon-3-carbonsäureamid in 100 ml Tetrahydrofuran mit 1.8 g Kaliumethylat versetzt und anschließend bei Raumtemperatur 30 Minuten gerührt. Nach Zugabe von 300 ml Ethylacetat wird zweimal mit je 50 ml 2 N Chlorwasserstoffsäure und anschließend mit Wasser gewaschen, getrocknet und im Vakuum eingedampft. Das Rohprodukt wird mit einer Mischung aus Ethylacetat und Hexan als Eluant an einer Silicagelsäule chromatographiert. Man erhält 3.8 g *(R)*-(--)-5-(3-Ethoxy-4-methoxyphenyl)-5-methyl-2-oxazolidinon. Nach dem Umkristallisieren aus Hexan-Ethylacetat verbleiben 3.1 g. Schmelzpunkt 87 °C. $[\alpha]_D$ = -51° ($CHCl_3$).

***(S)*-(+)-5-(3-Ethoxy-4-methoxyphenyl)-5-methyl-2-oxazolidinon**

Unter Eiskühlung wird eine Lösung von 10.1 g N-[*(R)*-1-(1-Naphthyl)ethyl]-*(S)*-5-(3-ethoxy-4-methoxyphenyl)-5-methyl-2-oxazolidinon-3-carbonsäureamid in 200 ml Tetrahydrofuran mit 3.6 g Kaliumethylat versetzt und anschließend 16 Stunden bei Raumtemperatur gerührt. Nach Zugabe von 400 ml Ethylacetat wird zweimal mit je 50 ml 2 N Chlorwasserstoffsäure und anschließend mit Wasser gewaschen, getrocknet und im Vakuum eingedampft. Das Rohprodukt von 11.5 g wird mit einer Mischung aus Ethylacetat und Hexan als Eluant an einer Silicagelsäule chromatographiert. Man erhält 5.5 g *(S)*-(+)-5-(3-Ethoxy-4-methoxypheny])-5-methyl-2-oxazolidinon. Nach Umkristallisation aus Hexan-Ethylacetat verbleiben 4.1 g. Schmelzpunkt 85 °C. $[\alpha]_D$ = +49° ($CHCl_3$).

**Beispiel 4**

**Trennung der Diastereomeren von *(R,S)*-5-(3-Benzyloxy-4-methoxyphenyl)-5-methyl-2-oxazolidinon**

3 g *(R,S)*-5-(3-Benzyloxy-4-methoxyphenyl)-5-methyl-2-oxazolidinon - analog hergestellt wie *(R,S)*-5-(3-Cyclopentyloxy-4-methoxyphenyl)-5-methyl-2-oxazolidinon - werden an einer Chiraphersäule (25 µm) in einer Procrom-Anlage mit einem Hexan-Dioxan-Gemisch chromatographiert. Es werden 1.2 g *(S)*-(+)-5-(3-Benzyloxy-4-methoxyphenyl)-5-methyl-2-oxazolidinon, Schmelzpunkt 116.8 °C, $[\alpha]_D$ = +38.9° ($CHCl_3$), sowie 1.1 g *(R)*-(--)-5-(3-Benzyloxy-4-methoxyphenyl)-5-methyl-2-oxazolidinon, Schmelzpunkt 116.7 °C, $[\alpha]_D$ = +38.4° ($CHCl_3$) eluiert.

***(R)*-(--)-5-(3-Hydroxy-4-methoxyphenyl)-5-methyl-2-oxazolidinon**

1.1 g *(R)*-(--)-5-(3-Benzyloxy-4-methoxyphenyl)-5-methyl-2-oxazolidinon werden in 40 ml Ethylacetat gelöst und mit 100 mg Palladium/Kohle 10%ig versetzt. Es wird bis zur Beendigung der Wasserstoffaufnahme hydriert. Nach Filtration über Kieselgel und Eindampfen im Vakuum erhält man 750 mg (R)-(--)-5-(3-Hydroxy-4-methoxyphenyl)-5-methyl-2-oxazolidinon, Schmelzpunkt 141.6 °C. $[\alpha]_D$ = -28.2° ($CHCl_3$).

***(R)*-(--)-5-(3-Cyclobutyloxy-4-methoxyphenyl)-5-methyl-2-oxazolidinon**

Eine Lösung von 80 mg (R)-(--)-5-(3-Hydroxy-4-methoxyphenyl)-5-methyl-2-oxazolidinon werden in 1 ml Dimethylformamid wird mit 25 mg Natriumhydrid (55 - 65%ig) versetzt und 15 Minuten bei 60 °C gerührt. Nach dem Abkühlen wird 0.04 ml Bromcyclobutan zugetropft und 2 Stunden bei 110 °C gerührt. Das Reaktionsgemisch wird im Ölvacuum am Kugelrohr zur Trockne eingeengt. Der Rückstand wird durch Chromatographie an einer Kieselgelsäule, mit einem Hexan-Ethylacetat-Gemisch als Eluant gereinigt. Es resultieren 52 mg *(R)*-(--)-5-(3-Cyclobutyloxy-4-methoxyphenyl)-5-methyl-2-oxazolidinon, Schmelzpunkt 132.5 °C. $[\alpha]_D$= -38.6° ($CHCl_3$).

**Beispiel 5**

***(R)*-(--)-5-(3-Isobutenyloxy-4-methoxyphenyl)-5-methyl-2-oxazolidinon**

Eine Lösung von 710 mg *(R)*-(--)-5-(3-Hydroxy-4-methoxyphenyl)-5-methyl-2-oxazolidinon in 30 ml Ethanol wird nacheinander mit 658 mg Kaliumcarbonat und 0.48 ml Methallychlorid versetzt. Nach 15 Stunden Rühren bei 70 °C wird filtriert und die Lösung im Vakuum eingedampft. Der ölige Rückstand wird durch Chromatographie an einer Kieselgelsäule mit einem Hexan-Ethyl-acetat-Gemisch als Eluant gereinigt. Es resultieren 620 mg *(R)*-(--)-5-(3-Isobutenyloxy-4-methoxyphenyl)-5-methyl-2-oxazolidinon, ölig. $[\alpha]_D$ = -24.3°.

**Beispiel 6**

***(R)*-(--)-5-(3-Isobutyloxy-4-methoxyphenyl)-5-methyl-2-oxazolidinon**

360 mg *(R)*-(--)-5-(3-Isobutenyloxy-4-methoxyphenyl)-5-methyl-2-oxazolidinon werden in 10 ml Ethylacetat gelöst und mit 50 mg Palladium/Kohle (10%ig) versetzt. Es wird bis zur Beendigung der Wasserstoffaufnahme hydriert. Nach Filtration über Kieselgur und Eindampfen im Vakuum erhält man einen öligen Rückstand. Das Rohprodukt wird durch Chromatographie an einer Kieselgelsäule, mit einem Hexan-Aceton-Gemisch als Eluant, gereinigt. Es resultieren 186 mg *(R)*-(--)-5-(3-Isobutyloxy-4-methoxyphenyl)-5-methyl-2-oxazolidinon, Schmelzpunkt 93.7 °C $[\alpha]_D$ = -24.7°.

## Patentansprüche

1. *(R)*-(--)-Methylphenyloxazolidinon-Derivate der Formel I,

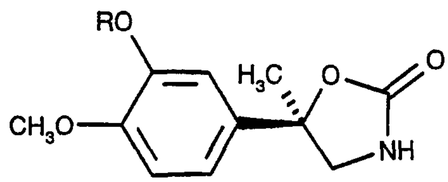

(I)

worin R einen Kohlenwasserstoffrest mit bis zu 5 C-Atomen bedeutet.

2. *(R)*-(--)-5-(3-Cyclopentyloxy-4-methoxyphenyl)-5-methyl-2-oxazolidinon
*(R)* -(--)-5-(3-Ethoxy-4-methoxyphenyl)-5-methyl-2-oxazolidinon
*(R)*-(--)-5-(4-Methoxy-3-propoxyphenyl)-5-methyl-2-oxazolidinon
*(R)*-(--)-5-(3-Cyclobutyloxy-4-methoxyphenyl)-5-methyl-2-oxazolidinon
*(R)*-(--)-5-(3-Isobutenyloxy-4-methoxyphenyl)-5-methyl-2-oxazolidinon
*(R)*-(--)-5-(3-Isobutyloxy-4-methoxyphenyl)-5-methyl-2-oxazolidinon
nach Anspruch 1.

3. Arzneimittel enthaltend eine Verbindung nach Anspruch 1 oder 2.

4. Verwendung der Verbindungen nach Anspruch 1 oder 2 zur Herstellung eines Arzneimittels zur Behandlung von Krankheiten, die durch die Aktivierung des Tumornekrosefaktors vermittelt werden.

5. Verwendung der Verbindungen nach Anspruch 1 oder 2 zur Herstellung eines Arzneimittels zur Behandlung Multipler Sklerose.

6. Verfahren zur Herstellung der Verbindungen nach Anspruch 1 dadurch gekennzeichnet, daß man deren Racemat mit einem optisch aktiven Hilfsstoff in das Diastereomerengemisch überführt und anschließend den optisch aktiven Hilfsstoff abtrennt.

7. Verfahren zur Herstellung der Verbindungen nach Anspruch 1 dadurch gekennzeichnet, daß man deren Racemat an chiralen Säulen chromatographiert.

8. Verfahren zur Herstellung der Verbindungen nach Anspruch 1 dadurch gekennzeichnet, daß man *(R)*-(--)-5-(3-Hydroxy-4-methoxyphenyl)-5-methyl-2-oxazolidinon verethert und gewünschtenfalls anschließend R in der Bedeutung Alkenyl reduziert.

## Claims

1. (R)-(-)-Methylphenyloxazolidinone derivatives of the formula I

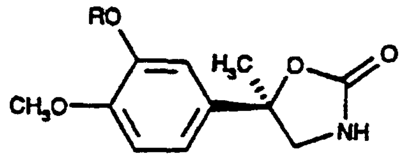

(I)

in which R denotes a hydrocarbon radical with up to 5 C atoms.

2. (R)-(-)-5-(3-Cyclopentyloxy-4-methoxyphenyl)-5-methyl-2-oxazolidinone
(R)-(-)-5-(3-ethoxy-4-methoxyphenyl)-5-methyl-2-oxazolidinone
(R)-(-)-5-(4-methoxy-3-propoxyphenyl)-5-methyl-2-oxazolidinone
(R)-(-)-5-(3-cyclobutyloxy-4-methoxyphenyl)-5-methyl-2-oxazolidinone
(R)-(-)-5-(3-isobutenyloxy-4-methoxyphenyl)-5-methyl-2-oxazolidinone
(R)-(-)-5-(3-isobutyloxy-4-methoxyphenyl)-5-methyl-2-oxazolidinone
according to Claim 1.

3. Pharmaceutical containing a compound according to Claim 1 or 2.

4. Use of the compounds according to Claim 1 or 2 for producing a pharmaceutical for the treatment of diseases mediated by activation of tumour necrosis factor.

5. Use of the compounds according to Claim 1 or 2 for producing a pharmaceutical for the treatment of multiple sclerosis.

6. Process for the preparation of the compounds according to Claim 1, characterized in that the racemate thereof is converted with an optically active ancillary substance into the diastereomer mixture and then the optically active ancillary substance is removed.

7. Process for the preparation of the compounds according to Claim 1, characterized in that the racemate thereof is chromatographed on chiral columns.

8. Process for the preparation of the compounds according to Claim 1, characterized in that (R)-(-)-5-(3-hydroxy-4-methoxyphenyl)-5-methyl-2-oxazolidinone is etherified and, if required, subsequently R meaning alkenyl is reduced.

**Revendications**

1. Dérivés de *(R)*-(-)-méthylphényloxazolidinone de formule I,

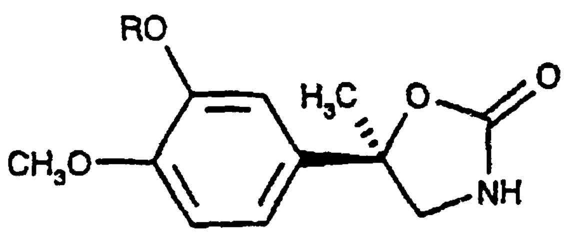

(I)

dans laquelle
R représente un radical hydrocarboné ayant jusqu'a 5 atomes de carbone.

2. *(R)*-(-)-5-(3-Cyclopentyloxy-4-méthoxyphényl)-5-méthyl-2-oxazolidinone
*(R)*-(-)-5-(3-éthoxy-4-méthoxyphényl)-5-méthyl-2-oxazolidinone
*(R)*-(-) -5- (4-méthoxyphényl-3-propoxy)-5-méthyl-2-oxazolidinone

*(R)-(-)-5-(3-cyclobutyloxy-4-méthoxyphényl)-5-méthyl-2-*oxazolidinone
*(R)*-(-)-5-(3-isobuténylоxy-4-méthoxyphényl)-5-méthyl-2-oxazolidinone
*(R)*-(-)-5- (3-isobutyloxy-4-méthoxyphényl)-5-méthyl-2-oxazolidinone
selon la revendication 1.

**3.** Médicament contenant un composé selon la revendication 1 ou 2.

**4.** Utilisation des composés selon la revendication 1 ou 2 pour l'élaboration d'un médicament destiné au traitement de troubles qui sont induits par l'activation du facteur de nécrose tumorale.

**5.** Utilisation des composés selon la revendication 1 ou 2 pour l'élaboration d'un médicament destiné au traitement de la sclérose en plaques.

**6.** Procédé de préparation des composés selon la revendication 1, caractérisé en ce que l'on transforme leur racémate en mélange de diastéréo-isomères à l'aide d'un auxiliaire optiquement actif et on sépare ensuite l'auxiliaire optiquement actif.

**7.** Procédé de préparation des composés selon la revendication 1, caractérisé en ce que leur racémate est soumis à une chromatographie sur des colonnes chirales.

**8.** Procédé de préparation des composés selon la revendication 1, caractérisé en ce que l'on éthérifie la *(R)*-(-)-5-(3-hydroxy-4-méthoxyphényl)-5-méthyl-2-oxazolidinone et ensuite, si on le souhaite, on réduit R en alcényle.